# EUROPEAN PATENT SPECIFICATION

(11) **EP 0 502 016 B1**
(45) Date of publication and mention of the grant of the patent: **02.10.1996**
(21) Application number: 90917062.3
(22) Date of filing: 26.11.1990
(51) Int. Cl.: C12N 15/31, C07K 14/235, C12P 21/02, A61K 39/10

(54) **NOVEL VACCINE**
NEUER IMPFSTOFF
NOUVEAU VACCIN

(30) Priority: 29.11.1989 US 442808; 22.12.1989 US 455648
(43) Date of publication of application: 09.09.1992
(73) Proprietor: SMITHKLINE BEECHAM BIOLOGICALS s.a., B-1330 Rixensart (BE)
(72) Inventor: LOCHT, Camille, F-59100 Roubaix (FR); LOBET, Yves, B-1332 Genval (BE)
(74) Representative: White, Susan Mary
(86) International application number: EP9002034
(87) International publication number: WO9108294

(56) References cited:
- EP-A- 0 322 115
- Infection and Immunity, vol. 57, no. 12, December 1989, American Society for Microbiology, M.A. Schmidt et al.: "Inhibition of pertussis toxin binding to model receptors by antipeptide antibodies directed at an antigenic domain of the S2 subunit", pages 3828-3833
- Pathogenesis and Immunity in Pertussis, vol. 57, chapter 6, John Wiley 6 Sons Ltd., Michio Ui: "The multiple biological activities of pertussis toxin", pages 121-145
- Science, vol. 246, 27 October 1989, M. Pizza et al.: "Mutants of pertussis toxin suitable for vaccine development", pages 497-500
- Infection and Immunity, vol. 56, no. 8, August 1988, American Society for Microbiology, J.T. Barbieri et al.: "ADP-Ribosyltransferase mutations in the catalytic S-1 subunit of pertussis toxin", pages 1934-1941
- The Journal of Biological Chemistry, vol. 264, no. 11, 15 April 1989, The American Society for Biochemistry and Molecular Biology, Inc., (US); H.R. Kaslow et al.: "Alkylation of cysteine 41, but not cysteine 200, decreases the ADP-ribosyltransferase activity of the S1 subunit of pertussis toxin", pages 6386-6390
- Infection and Immunity, vol. 55, no. 5, May 1987, American Society for Microbiology, G.D. Armstrong et al.: "Maintenance of biological activity of pertussis toxin radioiodinated while bound to fetuinagarose", pages 1294-1299
- Infection and Immunity, vol. 55, no. 11, November 1987, American Society for Microbiology, C. Locht et al; pages 2546-2553
- Biotechnology, vol. 6, no. 6, June 1988, (New York, US); W. Neal Burnette et al.: "Direct expression of Bordetella pertussis toxin subunits to high levels in Escherichia coli", see pages 699-705

## Description

### Field of the Invention

This invention relates to genetic modifications of the Bordetella pertussis toxin and to a vaccine comprising an immunoprotective amount of such protein.

### Background of the Invention

The members of the genus Bordetella are pathogenic microorganisms involved in the infection of the respiratory tract. The genus is comprised of four species; B. pertussis, B. parapertussis, B. bronchiseptica, and B. avium. The most virulent species to man is B. pertussis, which is the etiologic agent of whooping cough.

Current conventional pertussis vaccines contain whole but inactivated B. pertussis cells. Such cells are inactivated by treatment at 56°C for 30 minutes and/or treatment with formaldehyde. In spite of inactivation, such whole cell vaccines retain a substantial amount of toxicity.

As a result, alternate pertussis vaccines are available which are prepared from avirulent or toxin-deficient strains of B. pertussis. However, these vaccines have proven to be much less protective than those prepared from virulent strains. See, for example, Wardlaw et al., J Med Microbiol 9:89-100 (1976).

B. pertussis produces a number of toxins (pertussis toxin, adenylate cyclase, dermonecrotic toxin, and tracheal cytotoxin) which destroy the clearance mechanisms of the respiratory tract, or interfere with the immune response (F. Mooi, Antonie van Leeuwenhoek, 54:465-474 (1988)). A wide variety of biological activities, such as histamine sensitization, insulin secretion, lymphocytosis promotion and immunopotentiating effects can be attributed to the pertussis toxin (J. Munoz, in Pertussis Toxin, p. 1-18, Sekura et al., Eds., Academic Press, New York, 1985). In addition, it has been shown that the administration of the B. pertussis toxin in mice protected them against subsequent challenge (Munoz et al., Infect Immun 32:243-250 (1981)). Pertussis toxin is therefore an important constituent in a vaccine against whooping cough and is included in the acellular component vaccines being tested and used in several countries (Sato et al., Lancet, 1984-I:122 (1984)). Paradoxically, the pertussis toxin, which is capable of eliciting an immune response, may itself be responsible for the harmful side effects associated with current vaccines (Steinman et al., Proc. Natl Acad Sci USA, 82:8733 (1985)). These harmful effects can range from simple flushing to permanent neurological damage and in some instances, death.

The pertussis toxin is composed of five different subunits, designated S1 to S5 based on their electrophoretic migration in SDS-polyacrylamide gels. The subunits associate in the molar ratio of 1:1:1:2:1, respectively, to form the holotoxin. Functionally, the pertussis toxin can be divided into the A protomer, or S1 subunit, which contains adenosine diphosphate (ADP)-ribosylation activity, and the B-oligomer, comprised of subunits S2 through S5, which contains target cell receptor binding activities. Thus the B-oligomer is essential in bringing the A protomer into contact with the target-cell's membrane.

Locht et al., Science 232: 1258-64 (1986), disclose that the subunits of the pertussis toxin are encoded by closely linked cistrons. Locht et al. further disclose the nucleotide sequence of the B. pertussis toxin gene and the amino acid sequences for the individual subunits.

Locht et al., NAR 14:3251-61 (1986), reveal the cloning of a 4.5 kb DNA fragment from the B. pertussis toxin gene containing at least the S4 subunit and a portion of another subunit gene. Sequence analysis revealed that the mature S4 subunit is derived by proteolytic cleavage of a precursor molecule.

Nicosia et al., Infect Immun 55:963-7 (1987), disclose expression of each of the five B. pertussis toxin subunits as fusions to DNA polymerase MS2. Antisera raised to these proteins were found not to be immunoprotective in vivo or in vitro.

Locht et al., Infect Immun 55:2546-2553 (1987), disclose the expression of the S1 and S2 subunits of pertussis toxin in E. coli as fusions to 6 amino acid residues of beta-gacactosidase followed by 5 amino acids encoded by a polylinker. It was disclosed that the recombinant S1 subunit displayed enzymatic activities. A truncated version of the S1 subunit was disclosed in which the last 48 amino acid residues, i.e., the carboxy terminus, was deleted.

Sclavo SpA, EP-A-232,229, published August 12, 1987, disclose the cloning and expression of a B. Pertussis toxin gene, which contains subunits S1 through S5 in E. coli.

Bellini et al., EP-A-281,530, published September 7, 1988, disclose expression of mature B. pertussis subunits in B. subtilis

Burnette et al., EP-A-306,318, published March 8, 1989, report the subcloning and expression of individual B. pertussis toxin subunits in E. coli. Burnette et al. disclose that the S4 subunit could only be expressed upon removal of the signal peptide coding sequence. Burnette et al. also disclose S1 subunit analogs expressed in E. coli with modifications between amino acids Val⁷ to Pro¹⁴.

Burns et al., U.S. Patent 4,845,036, disclose a method for isolating the wild-type B. pertussis B-oligomer (i.e., subunits S2-S5) by dissociation of the holotoxin (i.e., subunits S1-S5).

Sato et al., EP-A-296,765, published December 28, 1988, disclose B. pertussis variants which produce mutant pertussis toxin proteins. The variants arose from exposure of virulent B. pertussis with nitrosoguanidine, a known mutagen.

M. Ui, (in Pathogenesis and Immunity in Pertussis, Wardlaw et al., eds., p.121-145, Wiley & Sons, Chichester, 1988) discloses that certain chemical modifications, e.g., acylation, of the pertussis toxin lysine residues eliminate all biological activity. Methylation of the pertussis toxin, which also modifies lysine residues, does not affect the ADP-ribosylation activity but does reduce or abolish certain biological activities associated with the B-oligomer, for example, mitogenic activity, stimulation of glucose oxidation, promotion of lymphocytosis and histamine-sensitizing activity. Ui further discloses that methylation of dimer D2 (i.e., pertussis toxin subunits S3-S4), but not dimer D1 (i.e., pertussis toxin. subunits S2-S4) or subunit S5, eliminates the mitogenic activity associated with the B-oligomer. There is no disclosure or suggestion, however, as to which specific regions or specific lysine residues of the B-oligomer are involved in the methylation or acylation.

Hausman et al., Infect Immun 57:1760-64 (1989), disclose immunization of mice with the pertussis toxin dimeric subunits, D1 (i.e., S2-S4) and D2 (i.e., S3-S4). The antisera raised to these dimers were able to recognize B. pertussis toxin and neutralize its toxic effects in vitro.

Capiau et al., U.S. Patent Application Serial Number 07/222,991,*filed July 22, 1988 disclose modification of the B. pertussis toxin S1 subunit at amino acid position 26 (i.e., tryptophan). This residue can be modified either chemically or by site-directed mutagenesis to substantially inactivate the enzymatic activity of the S1 subunit.
* EP - A - 0 352 250

Bellini et al., Gene, 69:325-330 (1988), recite a general method for site-directed mutagenesis for double-stranded plasmid DNA. Bellini et al. disclose that their method is particularly valuable where long deletions are needed. Exemplified is the deletion of the B. pertussis S2 subunit signal sequence coding region located on an E. coli - B. subtilis shuttle vector.

Black et al., EP-A-275, 689, published July 27, 1988 and Infect Immun 55:2465-70 (1987), disclose expression of the S4 subunit in E. coli. In addition, Black et al. disclose mutations in the B. pertussis toxin gene that were either deletions generated by Bal31 exonuclease or insertions with the kanamycin resistance gene. These mutations were then introduced by allelic exchange into the B. pertussis chromosome.

Klein et al., EP-A-322,115, published June 28, 1989, disclose substitution mutations of the B. pertussis toxin. Klein et al. also disclose deletion mutations of the S1 subunit. However, of the deletion mutations disclosed, only one mutation, Glu¹²⁹, was weakly reactive against antibodies to the S1 subunit.

It is an object of this invention to provide an improved B. pertussis vaccine comprising a modified B. pertussis toxin or subunits thereof which are immunogenic, yet non-toxic.

### Summary of Invention

The present invention relates to a recombinant DNA molecule which encodes a protein specifically reactive with antibodies against the wild-type pertussis toxin but which is defective in pertussis toxin target cell receptor binding.

In related aspects, this invention is a recombinant plasmid which comprises the recombinant DNA molecule of this invention operatively linked to a regulatory region. Said regulatory region contains regulatory sequences necessary for transcription of the protein coding sequence and subsequent translation in a host cell transformed with the recombinant plasmid of the invention.

This invention also relates to a pertussis toxin protein encoded by the recombinant DNA molecule of the invention, which is specifically reactive with antibodies against the wild-type pertussis toxin but which is also defective in pertussis toxin target cell receptor binding.

In another aspect, this invention is a vaccine for stimulating protection against whooping cough wherein such vaccine comprises an immunoprotective and non-toxic quantity of the protein encoded by the recombinant DNA molecule of the invention.. For vaccinal purposes, the protein of the invention may be purified away from the host cell or cell culture medium, or alternatively, it may be associated with the outer surface membrane of the host cell.

This invention further relates to a process for preparing the protein of the invention. This process comprises growing a host cell transformed with the recombinant DNA molecule of this invention in a suitable culture medium.

### Detailed Description of the Invention

The present invention is based on the discovery that modification of one or more of the naturally occurring amino acid residues of the Bordetella pertussis toxin B-oligomer DNA coding sequence greatly reduces the binding of pertussis toxin to target cell receptors thereby greatly reducing the toxicity of the pertussis toxin while retaining its ability to elicit a protective immunogenic response.

Many, but not all of the biological activities of the pertussis toxin (PT) are the result of three essential molecular steps. The first step involves the binding of PT to the receptors on the target cell membranes via the B-oligomer (subunits S2 through S5). Next, the S1 subunit must translocate into the cytoplasm of the target cell. Finally, the internalized S1 subunit has to express its enzymatic activity which includes NAD-glycohydrolysis and ADP-ribosylation. (For a review, see Ui, M., in Pathogenesis and Immunity in Pertussis, p. 121-145, Wardlaw and Parton, eds., Wiley and Sons, Chichester, 1988). Elimination of any of these three steps drastically diminishes the biological activities of said toxin. Therefore, a. modified pertussis toxin which is incapable of binding to target cell receptors, drastically reduces or eliminates the toxic activities associated with the pertussis toxin.

The B-oligomer, which is associated with adherence or binding of PT to host cells, is composed of subunits S2-S5. The B-oligomer can be further divided into two dimers, D1 and D2, which are connected by subunit S5. Dimer D1 comprises subunits S2 and S4, whereas dimer D2 comprises subunits S3 and S4 (see, Tamura et al., Biochemistry, 21:5516-22 (1982)). Each dimer specifically interacts with different target cell receptor molecules. For example, D1 appears to be responsible for the binding of PT to such glycoproteins as haptoglobin or fetuin (see, Francotte et al., in Vaccine 89, p. 243-247, Lerner et al., eds., Cold Spring Harbor Laboratory, Cold Spring Harbor, NY, 1989), whereas D2 can specifically bind to Chinese Hamster Ovary (CHO) cell membranes (see, Brennan et al., J Biol Chem, 263:4895-99 (1988)). It is presumed that this binding specificity is attributed to the structural differences between S2 and S3.

The nucleotide sequence for the Bordetella pertussis toxin gene is disclosed by Locht et al. (Science, 232:1258-64 (1986)): 
1 GAATTCGTCG CCTCGCCCTG GTTCGCCGTC ATGGCCCCCA AGGGAACCGA

51 CCCCAAGATA ATCGTCCTGC TCAACCGCCA CATCAACGAG GCGCTGCAGT

101 CCAAGGCGGT CGTCGAGGCC TTTGCCGCCC AAGGCGCCAC GCCGGTCATC
 
151 GCCACGCCGG ATCAGACCCG CGGCTTCATC GCAGACGAGA TCCAGCGCTG
201 GGCCGGCGTC GTGCGCGAAA CCGGCGCCAA GCTGAAGTAG CAGCGCAGCC
251 CTCCAACGCG CCATCCCCGT CCGGCCGGCA CCATCCCGCA TACGTGTTGG
301 CAACCGCCAA CGCGCATGCG TGCAGATTCG TCGTACAAAA CCCTCGATTC
351 TTCCGTACAT CCCGCTACTG CAATCCAACA CGGCATGAAC GCTCCTTCGG
401 CGCAAAGTCG CGCGATGGTA CCGGTCACCG TCCGGACCGT GCTGACCCCC
451 CTGCCATGGT GTGATCCGTA AAATAGGCAC CATCAAAACG CAGAGGGGAA
501 GACGGGATGC GTTGCACTCG GGCAATTCGC CAAACCGCAA GAACAGGCTG
551 GCTGACGTGG CTGGCGATTC TTGCCGTCAC GGCGCCCGTG ACTTCGCCGG
601 CATGGGCCGA CGATCCTCCC GCCACCGTAT ACCGCTATGA CTCCCGCCCG
651 CCGGAGGACG TTTTCCAGAA CGGATTCACG GCGTGGGGAA ACAACGACAA
701 TGTGCTCGAC CATCTGACCG GACGTTCCTG CCAGGTCGGC AGCAGCAACA
751 GCGCTTTCGT CTCCACCAGC AGCAGCCGGC GCTATACCGA GGTCTATCTC
801 GAACATCGCA TGCAGGAAGC GGTCGAGGCC GAACGCGCCG GCAGGGGCAC
851 CGGCCACTTC ATCGGCTACA TCTACGAAGT CCGCGCCGAC AACAATTTCT
901 ACGGCGCCGC CAGCTCGTAC TTCGAATACG TCGACACTTA TGGCGACAAT
951 GCCGGCCGTA TCCTCGCCGG CGCGCTGGCC ACCTACCAGA GCGAATATCT
1001 GGCACACCGG CGCATTCCGC CCGAAAACAT CCGCAGGGTA ACGCGGGTCT
1051 ATCACAACGG CATCACCGGC GAGACCACGA CCACGGAGTA TTCCAACGCT
1101 CGCTACGTCA GCCAGCAGAC TCGCGCCAAT CCCAACCCCT ACACATCGCG
1151 AAGGTCCGTA GCGTCGATCG TCGGCACATT GGTGCATGGC GCCGGTGATA
1201 GCGCTTGCAT GGCGCGGCAG GCCGAAAGCT CCGAGGCCAT GGCAGCCTGG
1251 TCCGAACGCG CCGGCGAGGC GATGGTTCTC GTGTACTACG AAAGCATCGC
1301 GTATTCGTTC TAGACCTGGC CCAGCCCCGC CCAACTCCGG TAATTGAACA
1351 GCATGCCGAT CGACCGCAAG ACGCTCTGCC ATCTCCTGTC CGTTCTGCCG
1401 TTGGCCCTCC TCGGATCTCA CGTGGCGCGG GCCTCCACGC CAGGCATCGT
 
1451 CATTCCGCCG CAGGAACAGA TTACCCAGCA TGGCAGCCCC TATGGACGCT
1501 GCGCGAACAA GACCCGTGCC CTGACCGTGG CGGAATTGCG CGGCAGCGGC
1551 GATCTGCAGG AGTACCTGCG TCATGTGACG CGCGGCTGGT CAATATTTGC
1601 GCTCTACGAT GGCACCTATC TCGGCGGCGA ATATGGCGGC GTGATCAAGG
1651 ACGGAACACC CGGCGGCGCA TTCGACCTGA AAACGACGTT CTGCATCATG
1701 ACCACGCGCA ATACGGGTCA ACCCGCAACG GATCACTACT ACAGCAACGT
1751 CACCGCCACT CGCCTGCTCT CCAGCACCAA CAGCAGGCTA TGCGCGGTCT
1801 TCGTCAGAAG CGGGCAACCG GTCATTGGCG CCTGCACCAG CCCGTATGAC
1851 GGCAAGTACT GGAGCATGTA CAGCCGGCTG CGGAAAATGC TTTACCTGAT
1901 CTACGTGGCC GGCATCTCCG TACGCGTCCA TGTCAGCAAG GAAGAACAGT
1951 ATTACGACTA TGAGGACGCA ACGTTCGAGA CTTACGCCCT TACCGGCATC
2001 TCCATCTGCA ATCCTGGATC ATCCTTATGC TGAGACGCTT CCCCACTCGA
2051 ACCACCGCCC CGGGACAGGG CGGCGCCCGG CGGTCGCGCG TGCGCGCCCT
2101 GGCGTGGTTG CTGGCATCCG GCGCGATGAC GCATCTTTCC CCCGCCCTGG
2151 CCGACGTTCC TTATGTGCTG GTGAAGACCA ATATGGTGGT CACCAGCGTA
2201 GCCATGAAGC CGTATGAAGT CACCCCGACG CGCATGCTGG TCTGCGGCAT
2251 CGCCGCCAAA CTGGGCGCCG CGGCCAGCAG CCCGGACGCG CACGTGCCGT
2301 TCTGCTTCGG CAAGGATCTC AAGCGTCCCG GCAGCAGTCC CATGGAAGTC
2351 ATGTTGCGCG CCGTCTTCAT GCAACAACGG CCGCTGCGCA TGTTTCTGGG
2401 TCCCAAGCAA CTCACTTTCG AAGGCAAGCC CGCGCTCGAA CTGATCCGGA
2451 TGGTCGAATG CAGCGGCAAG CAGGATTGCC CCTGAAGGCG AACCCCATGC
2501 ATACCATCGC ATCCATCCTG TTGTCCGTGC TCGGCATATA CAGCCCGGCT
2551 GACGTCGCCG GCTTGCCGAC CCATCTGTAC AAGAACTTCA CTGTCCAGGA
2601 GCTGGCCTTG AAACTGAAGG GCAAGAATCA GGAGTTCTGC CTGACCGCCT
2651 TCATGTCGGG CAGAAGCCTG GTCCGGGCGT GCCTGTCCGA CGCGGGACAC
2701 GAGCACGACA CGTGGTTCGA CACCATGCTT GGCTTTGCCA TATCCGCGTA
 
2751 TGCGCTCAAG AGCCGGATCG CGCTGACGGT GGAAGACTCG CCGTATCCGG
2801 GCACTCCCGG CGATCTGCTC GAACTGCAGA TCTGCCCGCT CAACGGATAT
2851 TGCGAATGAA CCCTTCCGGA GGTTTCGACG TTTCCGCGCA ATCCGCTTGA
2901 GACGATCTTC CGCCCTGGTT CCATTCCGGG AACACCGCAA CATGCTGATC
2951 AACAACAAGA AGCTGCTTCA TCACATTCTG CCCATCCTGG TGCTCGCCCT
3001 GCTGGGCATG CGCACGGCCC AGGCCGTTGC GCCAGGCATC GTCATCCCGC
3051 CGAAGGCACT GTTCACCCAA CAGGGCGGCG CCTATGGACG CTGCCCGAAC
3101 GGAACCCGCG CCTTGACCGT GGCCGAACTG CGCGGCAACG CCGAATTGCA
3151 GACGTATTTG CGCCAGATAA CGCCCGGCTG GTCCATATAC GGTCTCTATG
3201 ACGGTACGTA CCTGGGCCAG GCGTACGGCG GCATCATCAA GGACGCGCCG
3251 CCAGGCGCGG GGTTCATTTA TCGCGAAACT TTCTGCATCA CGACCATATA
3301 CAAGACCGGG CAACCGGCTG CGGATCACTA CTACAGCAAG GTCACGGCCA
3351 CGCGCCTGCT CGCCAGCACC AACAGCAGGC TGTGCGCGGT ATTCGTCAGG
3401 GACGGGCAAT CGGTCATCGG AGCCTGCGCC AGCCCGTATG AAGGCAGGTA
3451 CAGAGACATG TACGACGCGC TGCGGCGCCT GCTGTACATG ATCTATATGT
3501 CCGGCCTTGC CGTACGCGTC CACGTCAGCA AGGAAGAGCA GTATTACGAC
3551 TACGAGGACG CCACATTCCA GACCTATGCC CTCACCGGCA TTTCCCTCTG
3601 CAACCCGGCA GCGTCGATAT GCTGAGCCGC CGGCTCGGAT CTGTTCGCCT
3651 GTCCATGTTT TTCCTTGACG GATACCGCGA ATGAATCCCT TGAAAGACTT
3701 GAGAGCATCG CTACCGCGCC TGGCCTTCAT GGCAGCCTGC ACCCTGTTGT
3751 CCGCCACGCT GCCCGACCTC GCCCAGGCCG GCGGCGGGCT GCAGCGCTGT
3801 CAACCACTTC ATGGCGAGCA TCGTGGTCGT ACTGCCGCGG CGGTCAGTGG
3851 CCACGGTGAC CATCGCCATA ATCTGGGCGG GCTACAAGCT GCTGTTCCGG
3901 CACGCCGATG TGCTGGACGT GGTGCGTGTG GTGCTGGCGG GAGCTGCTGA
3951 TCGGCGCATC GGCCGAAATC GCTCGTTATC TGCTGACCTG AATCCTGGAC
4001 GTATCGAACA TGCGTGATCC GCTTTTCAAG GGCTGCACCC GGCGCCGCGA
 
4051 TGCTGATGGC GTACCCGCCA CGGCAGGCCG TGTGCAGCCG GCACCATTCC

4101 CTGCTGGGCC ATCTCGGTTC AGCATCCGCT TTCTGGCCTT GTTTCCCGTG

4151 GCATTGCTGG CGATGCGGAT CATGATCCGG CGCGATGACC AGCAGTTCCG

4201 CCTGATC
 Wherein: the S1 subunit is encoded by nucleotides 507 to 1310; the mature S1 subunit is encoded by nucleotides 609 to 1310. The S2 subunit is encoded by nucleotides 1353 to 2030; the mature S2 subunit is encoded by nucleotides 1434 to 2030. The S4 subunit is encoded by nucleotides 2090 to 2482; the mature S4 subunit is encoded by nucleotides 2153 to 2482. The S5 subunit is encoded by nucleotides 2497 to 2856; the mature S5 subunit is encoded by nucleotides 2557 to 2856. The S3 subunit is encoded by nucleotides 2942 to 3622; the mature S3 subunit is encoded by nucleotides 3026 to 3622.

S2 and S3 are 70% homologous in their amino acid sequences and 75% homologous in their nucleotide sequences (see, Locht et al., Science, 232:1258-64 (1986)). Despite their homology, S2 and S3 cannot substitute for each other in the functionally active pertussis toxin (see, Tamura et al., supra). In addition, each of the S2 and S3 subunits bind to one S4 subunit. Hence the two dimers, D1 and D2, must be closely related to each other though their roles in B-oligomer function are quite distinct.

Chemical modification of the pertussis toxin can affect its biological activities. Acylation, for example, eliminates all biological activity associated with the pertussis toxin due to disruption of the quaternary structure (see, Nogimori et al., Biochim Biophys Acta, 801:220-231 (1984)). Methylation of the pertussis toxin, which modifies lysine residues, does not affect the ADP-ribosylation activity but does reduce or abolish activity associated with the B-oligomer (see Ui, M., in Pathogenesis and Immunity in Pertussis, p. 121-145, Wardlaw and Parton, eds., Wiley and Sons, Chichester, 1988). Furthermore, Ui discloses that methylation of dimer D2, but not dimer D1 or subunit S5, eliminates the mitogenic activity associated with the B-oligomer. Therefore, Ui concludes that the lysine residues play a role in the attachment of.D2 to the host cell's surface, but not the attachment of D1.

It has also been shown that iodination of pertussis toxin severely reduces some biological activities, such as hemagglutination and CHO cell clustering. See, Armstrong et al., Infect Immun, 55:1294-99 (1987). Armstrong et al. also disclose a method to radioiodinate the wild-type pertussis toxin in the presence of fetuin-agarose and still retain biological activity. It was reported that by using such method, all of the PT subunits were iodinated. However, Armstrong et al. report that the mechanism for the observed reduction in biological activity is not known.

The present invention thus describes a B. pertussis toxin DNA sequence which encodes a protein which is defective in target cell receptor binding activity and preferably lacks S1 subunit enzymatic activity as well, yet retains the capability to be recognized by anti-Pertussis Toxin antibodies.

Target cell receptor binding activity can be assayed by haptoglobin-binding as described by Francotte et al. (in Vaccine 89, p.243-247, Lerner et al., eds., Cold Spring Harbor Laboratory, Cold Spring Harbor, NY, 1989) or by CHO cell binding/cytotoxicity assays as described by Brennan et al. (J Biol Chem, 263:4895-99 (1988)) or Burns et al. (Infect Immun, 55:24 (1987)). S1 subunit enzymatic activity can be measured by the ADP-ribosylation assay as described by Burnette et al. (Science, 242:72 (1988)).

DNA molecules comprising the recombinant DNA molecule of this invention can be derived from any B. pertussis strain using known techniques, e.g., isolating the gene from a gene bank, making complementary or cDNAs from a mRNA template or via the polymerase chain reaction (see, U.S. Patent 4,800,159) or from isolates of clinical specimens. Alternatively., such recombinant DNA molecule may be synthesized by standard DNA synthesis techniques. Furthermore, various B. pertussis strains are publicly available from commercial depositories, e.g., from the American Type Culture Collection (ATCC), Rockville, Maryland, U.S.A.

As used herein, the term "DNA sequence which encodes a protein specifically reactive with antibodies against wild-type pertussis toxin but which is defective in pertussis toxin target cell receptor binding" means a DNA coding sequence which encodes a protein with decreased target cell binding activity relative to the wild-type pertussis toxin but still retains the ability to be recognized by anti-pertussis toxin antibodies, such as a coding sequence which encodes a protein comprising all the subunits (S1, S2, S3, S4 and S5) substantially as described by Locht et al. (Science 232:1258-64 (1986)) and all mutations or mutants thereof. The terms "mutations" or "mutants" as applied to the B oligomer or S2 or S3 subunits encompass any derivative of the recombinant DNA molecule of this invention which encodes a protein which is defective in target cell receptor binding and still retains the ability to be recognized by anti-Pertussis Toxin antibodies. Such mutations can be prepared by the deletion, addition, substitution, or rearrangement of amino acids and their nucleic acid coding sequences, or alternatively, by chemical modifications thereof.

Preferred embodiments of the recombinant DNA molecule of this invention include, but are not limited to, a recombinant DNA molecule containing amino acid deletions in Asn¹⁰⁵, or Tyr¹⁰², or Tyr¹⁰²⁻¹⁰³ of subunit S2, and/or Lys¹⁰, or Tyr⁹², or Lys⁹³, or Lys¹⁰⁵, or Tyr¹⁰², or Tyr¹⁰²⁻¹⁰³ of subunit S3, or single amino acid substitutions, e.g., Asn¹⁰⁵ to Asp of subunit S2.

The most preferred embodiments include, but are not limited to, deletions of Asn¹⁰⁵, or Tyr¹⁰², or Tyr¹⁰²⁻¹⁰³ in subunit S2 and/or Lys¹⁰⁵, or Tyr¹⁰², or Tyr^{102 103} in subunit S3.

Mutations in S1 (e.g., deletions of Trp²⁶, or His³⁵, or Ser⁴⁰, or Glu¹²⁹) are disclosed in U.S. Application Serial No. 07/381,888,* filed July 18, 1989, the entire disclosure of which is hereby incorporated by reference herein. Mutations of other S1 subunit amino acid residues (i.e., substitutions of Arg⁹, Arg¹³, Glu¹²⁹) are disclosed by Pizza et al., Science, 246:497-500 (1989) and EP-A-O 396 964,and also in EP-A-0306318. These references are incorporated by reference herein.

Preferably the DNA coding sequence of this invention will encode a protein that resembles the natural or wild-type protein as much as possible in tertiary structure, i.e., a protein which is able to be recognized by anti-pertussis toxin antibodies, yet is deficient in target cell receptor binding activity.

Other embodiments of the recombinant DNA molecule of this invention include a pertussis toxin coding sequence which encodes some, but not all of the subunits. Exemplary embodiments include, but are not limited to, D1 (S2 and 54), D2 (S3 and S4), S1-S2-S4, S1-S3-S4 and the B-oligomer (S2, S3, S4 and S5).

In another embodiment, the recombinant DNA molecule of the invention can be in the form of a hybrid, that is, a coding sequence which encodes a fusion polypeptide containing additional sequences which can carry one or more epitopes from other PT subunits, for example, [S2 epitope]-[S3 subunit], etc., other B. pertussis antigens, or other non-B. pertussis antigens. Alternatively, the recombinant DNA molecule of the invention can be fused to the DNA coding sequence of a carrier polypeptide which has immunostimulating properties, as in the case of an adjuvant, or which otherwise enhances the immune response to the B. pertussis toxin subunit(s), or which is useful in expressing, purifying or formulating the B. pertussis toxin subunit(s).

The recombinant DNA molecule of this invention may comprise additional DNA sequences, including e.g., a regulatory element, one or more selectable markers, and sequences that code for replication and maintenance functions. The regulatory region typically contains a promoter found upstream from the coding sequence of this invention, which functions in the binding of RNA polymerase and in the initiation of RNA transcription. In other words, the regulatory element or region is operatively linked to the coding sequence of this invention. It will be appreciated by one of skill in the art that the selection of regulatory regions will depend upon the host cell employed.

This invention also relates to a recombinant DNA plasmid comprising the recombinant DNA molecule of this invention.

Another aspect of this invention is a host cell transformed with the recombinant DNA molecule of this invention. Such host cell is capable of growth in a suitable culture medium and expressing the coding sequence of the invention. Such host cell is prepared by the method of this invention, i.e., by transforming a desired host cell with the plasmid of this invention. Such transformation is accomplished by utilization of conventional transformation techniques. Moreover, the recombinant DNA molecule of this invention can be integrated into the host cell's genome by conventional techniques, e.g., homologous recombination. The most preferred host cells of this invention include those belonging to the species E. coli and the genus Bordetella. Other host cells which may be suitable include, but are not limited to, mammalian cells, insect cells, yeast and other bacterial cells, e.g., Streptomyces, Bacillus and Salmomella. Thus, this invention and the product thereof is not limited to any specific host cell.

The present invention also relates to a protein encoded by the recombinant DNA molecule of this invention. Preferably such protein is produced by the transformed host cell of this invention, but such protein may be prepared by conventional peptide synthesis techniques.

The protein of this invention preferably has no more than about 50% of the haptoglobin binding or CHO cytotoxicity as compared to wild-type pertussis toxin. Most preferably, the protein of this invention has less than 10% and more preferably less than 5% of either of the assayed activities as compared to wild-type pertussis toxin.

The present invention also relates to a method of producing the protein encoded by the recombinant DNA molecule of this invention which comprises culturing the transformed host of the invention in an appropriate culture media and the isolation of such protein. By "appropriate culture media" is meant that media which will enable such host to express the coding sequence of the invention in recoverable quantity. It will be appreciated by one of skill in the art that the appropriate culture media to use will depend upon the host cell employed. The isolation of the protein so produced is accomplished from a culture lysate of the host, or if appropriate, directly from the host's culture medium, and such isolation is carried out by conventional protein isolation techniques. See, for example, Burns et al., U.S. Patent 4,845,036.

In a preferred embodiment of this invention, the coding sequence of the protein of the invention is expressed in a transformed B. pertussis host cell to produce an immunogenic yet substantially inactivated protein, i.e., a protein that is deficient in target-cell receptor binding, and in addition, may optionally be deficient in ADP- ribosyltransferase activity, but which is still specifically recognized by anti-Pertussis Toxin antibodies. In such a system, sequences that encode B. pertussis toxins are typically located on a suicide vector. Such suicide vector contains a sufficient amount of bacterial DNA to propagate the suicide vector in E. coli or some other suitable host. Such suicide vector also contains a sufficient amount of B. pertussis DNA flanking the toxin subunit coding sequence so as to permit recombination between a B. pertussis host deficient in the toxin gene and the heterologous toxin gene. It is understood to one skilled in the art that it is not essential to use a B. pertussis host deficient in the toxin gene, but that the absence of the toxin gene in the host prior to recombination will facilitate the screening and isolation of recombinant hosts which have incorporated the gene of interest. The recombinant B. pertussis arising from such homologous recombination are then selected by standard techniques. See, e.g., Stibitz et al., Gene 50:133-140 (1986).

The invention also encompasses a vaccine capable of inducing immunity against whooping cough. Such vaccine comprises an immunoprotective and non-toxic amount of the protein of the invention. Such vaccine typically contains 1-500 µg, preferably 5-25 µg of the protein of this invention, but is not limited to use of these amounts.

Further embodiments of the present invention include a whole cell vaccine for stimulating protection against whooping cough. Such vaccine comprises the protein of this invention expressed on the surface if transformed host cells of this invention. The transformed host cells are subsequently inactivated by conventional techniques to constitute an immunoprotective and non-toxic vaccine.

Other antigens which are known to be desirably administered in conjugation with pertussis toxin may also be included in the vaccine of this invention. Such additional components are known to those skilled in the art. Preferable additional components include tetanus toxoid and/or diptheria toxoid as well as filamentous hemagglutinin (FHA), agglutinogens 2 and 3, the 69 kD antigen, and/or any other protective antigen of B. pertussis.

The provision of such a vaccine thus allows for another aspect of the present invention, i.e., a method for immunizing a human against whooping cough which comprises administering the vaccine of the subject invention to such human.

The mode of administration of the vaccine of the invention may be any suitable route which delivers an immunoprotective amount of the protein of the subject invention to the host. However, the vaccine is preferably administered parenterally via the intramuscular or subcutaneous routes. Other modes of administration may also be employed, where desired, such as oral administration or via other parenteral routes, i.e., intradermally, intranasally or intravenously.

The vaccine of the invention may be prepared as a pharmaceutical composition containing an immunoprotective, non-toxic and sterile pharmaceutically acceptable carrier. In the vaccine of the invention, an aqueous solution of the protein of this invention can be used directly. Alternatively, the protein of this invention, with or without prior lyophilization, can be mixed together or with any of the various known adjuvants. Where the administration of the vaccine is parenteral, the protein of the invention can be optionally admixed or absorbed with any conventional adjuvant to enhance an immune response. Such adjuvants include among others, aluminum hydroxide, aluminum phosphate, muramyl dipeptide and saponins, such as Quil A. As a further exemplary alternative of the preparation of the vaccine of the invention, the protein of the invention can be encapsulated within microparticles such as liposomes. In yet another exemplary alternative of the preparation of the vaccine of the invention, the protein of the invention can be administered with an immunostimulating macromolecule, for example, tetanus toxoid. Alternatively, an aqueous suspension or solution containing the protein of the invention preferably is buffered at physiological pH. The protein of the invention may also be designed for oral digestion.

Vaccine preparation is generally described in New Trends and Developments in Vaccines, Voller et al. (eds.), University Park Press, Baltimore, Maryland, 1978. Encapsulation within liposomes is described by Fullerton, U.S. Patent 4,235,877. Conjugation of proteins to macromolecules is disclosed, for example, by Likhite, U.S. Patent 4,372,945 and Armor et al., U.S. Patent 4,474,757. Use of Quil A is disclosed by Dalsgaard et al., Acta Vet Scand 18:349 (1977).

It is preferred that the vaccine of the invention, when in a pharmaceutical preparation, be present in unit dosage forms. The appropriate prophylactically effective dose can be determined readily by those of skill in the art. The effective amount of protein contained in the vaccine of this invention may be in the range of effective amounts of antigen in conventional B. pertussis acellular or component vaccines, i.e., 5-25 µg of protein per unit dose. This dose may optionally be delivered with various amounts of filamentous hemagglutin (FHA) (approximately 10-25 µq per dose) and/or agglutinogens or other antigens. It will be understood, however, that the specific dose level for any particular patient will depend upon a variety of factors including the age, general health, the time of administration, the route of administration, synergistic effects with any other drugs being administered, and the degree of protection being sought. The administration can be repeated at suitable intervals if necessary.

The examples which follow are illustrative but not limiting of the present invention. Restriction enzymes and other reagents were used substantially in accordance with the vendors' instructions.

### EXAMPLES

Modification of pertussis toxin subunits by site-directed mutagenesis:

### Example 1. Mutagenesis of S2

Plasmid pPTX42, which encodes the complete nucleotide sequence for the pertussis toxin gene (Locht et al., Science, 232:1258-64 (1986)) was digested with the restriction enzymes XbaI and XmaI. A 750 base pair (bp) fragment was isolated and ligated into M13mp19 previously digested with XbaI and XmaI. The vector M13mp19, which can exist as a single-stranded or a double-stranded entity, is a standard subcloning vector suitable for site directed mutagenesis and is commonly available. See J. Messing, Meth Enzymol, 101:20-78 (1983). The resultant vector, designated φRIT20200, encodes the S2 subunit of pertussis toxin, plus 5' + 3' untranslated DNA. This vector was then used for subsequent mutagenesis experiments.

Site-directed mutagenesis was done according to the "Oligonucleotide-Directed in vitro Mutagenesis System" (Amersham, Arlington Heights, IL). A synthetic oligomer directed to the region of mutagenesis was annealed to the single stranded form of φRIT20200. This annealed oligomer was then used as a primer to replicate a vector which was the complement to φRIT20200 except for specific changes made in the synthetic oligomer region. Thus, one in the art is capable of rapidly inducing specific changes in a vector without affecting the remaining nucleic acid sequences. To delete amino acid 102 (tyrosine), i.e., Tyr¹⁰² of subunit S2, the following synthetic oligonucleotide primer was used: 5' CGTTGCTGTAGTGATCCGTT 3' This resulting vector, which is lacking the codon for amino acid 102 (i.e., Tyr) was designated φRIT20201. It was then used for subsequent mutagenesis experiments.

To delete tyrosine 103 (Tyr¹⁰³) from φRIT20201, the following mutagenesis primer was used: 5' TGACGTTGCTGTGATCCGTT 3' The resultant phage, lacking the codons for Tyr¹⁰² and Tyr¹⁰³, was designated φRIT20202. To ensure that the correct mutations had occurred and that no unwanted mutations were introduced into the S2 subunit gene, the S2 subunits of both mutant vectors were sequenced. The double stranded replicative form of phages φRIT20201 and φRIT20202 were then digested with XbaI and XmaI and the 750 bp fragments encoding the S2 mutations were inserted into the XbaI and XmaI sites of plasmid pRIT13070. Plasmid pRIT13070 (Capiau et al., U.S. Patent Application Serial Number 07/222,991* filed July 22, 1988) is a recombinant plasmid containing the complete pertussis toxin structural gene inserted into the ECoRI site of pUC7, which is a common cloning vector (Vieira et al., Gene, 19:259-68 (1982)).

The recombinant plasmids, containing the S2 mutations Tyr¹⁰² and Tyr¹⁰²⁻¹⁰³ inserted into the complete pertussis toxin structural gene, were designated pRIT13241 and pRIT13242 respectively. These plasmids were again sequenced to verify their modified nucleotide sequence.

To delete the S2 subunit codon asparagine 105 (Asn¹⁰⁵), the polymerase chain reaction (PCR) was used (Saiki et al., Science 230:1350-1354 (1985)); the PCR system is commercially available Perkin Elmer Cetus (Emeryville, California).

The mutagenesis of Asn¹⁰⁵ was performed as follows:
(a) Plasmid pRIT13070 was first denatured, then two synthetic oligonucleotides complementary to one strand of the denatured plasmid were annealed. The first oligonucleotide: (I) 5' CGTTCTAGACCTGGCCCAGCCCCG 3' encodes an XbaI restriction site 5' to the site to be mutagenized, the second oligonucleotide: (II) 5' TGGCGGTGACGCTGTAGTAG 3' spans the site to be mutagenized. The DNA between the two annealed oligonucleotides is then amplified via PCR.
(b) In the second step, two additional oligonucleotides, complementary to the opposite DNA stranc of step (a), were annealed to the denatured plasmid pRIT13070. One oligonucleotide: (III) 5' TGTCCCGGGGCGGTGGTTCGAGTG 3' encodes an XmaI restriction site 3' to the site to be mutagenized, and the other oligonucleotide: (IV) 5' CAGCGTCACCGCCACTCGCCTGCTCTCCAG 3' spans the site to be mutagenized and is complementary (i.e., capable of forming base pairs) with oligonucleotide (II) for a span of 14 nucleotides. The DNA between annealed oligonucleotides (III) and (IV) was then amplified via PCR. Finally, the two amplified DNA fragments were annealed to each other and amplification of the mutagenized DNA fragment between the XbaI and XmaI sites was carried out via PCR. This amplified DNA fragment was digested with the restriction enzymes XbaI and XmaI and then inserted into the XbaI and XmaI sites of an unmutagenized pRIT13070.

To substitute the S2 subunit codon asparagine (Asn¹⁰⁵) with aspartic acid (Asp), the PCR system was used. The procedure is the same as the deletion of Asn¹⁰⁵, disclosed above, except that different oligonucleotide sequences are used. Oligomers (II) and (IV) are replaced with oligomers (IIs) and IVs), respectively:

(IIs)   5'   TGGCGGTGACGTCGCTGTAGTAG   3'

(IVs) 5' CAGCGACGTCACCGCCACTCGCCTGCTCTCCAG 3'

After transformation, the recombinant mutant plasmids were analyzed by DNA sequence analysis to verify their modified nucleotide sequences.

### Example 2. Mutagenesis of S3

Digestion of pPTX42 (Locht et al., cited above) with the restriction enzyme PstI yielded, among others, a 960 bp fragment containing the coding region for the S3 subunit of the pertussis toxin. This fragment was isolated and ligated into the PstI site of M13mp9 (Messing et al., cited above). The recombinant phage, designated φRIT20300 was then used for mutagenesis experiments (Amersham system, supra) for the following S3 subunit mutations:

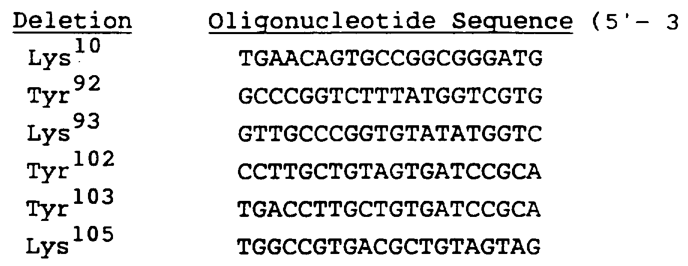

After mutagenesis, the site-specific mutants were sequenced to ensure that the correct mutation had been introduced. The double stranded replicative forms of the phage DNA were then isolated and digested with BglII and MluI. The 690 bp DNA fragments, containing the various S3 mutations, were then isolated.

A 2.7 kbp XmaI - EcoRI insert from plasmid pRIT13070 (cited above) was cloned into pUC9, a standard and commercially available cloning vector. This newly created plasmid, designated pPT3, was subsequently digested with BglII and MluI. Into this BglII - MluI site was ligated the 690bp BglII - MluI fragments from above, containing the various S3 mutations.

These recombinant plasmids, encoding for the various S3 mutations, were in turn digested with BamHI and BglII. The fragments, 1,750 bp in length, were isolated and ligated into the BamHI - BglII sites of pRIT13070. The various single S2 and S3 mutations are summarized in Table I below (mutations #1 to 9).

### Example 3. Mutagenesis of S1

Digestion of pPTX42 (Locht et al., cited above) with the restriction enzyme Sau3A yielded, among others, 560 bp fragment containing most of the coding region for the S1 pertussis toxin subunit, but lacking the carboxy terminus coding region. This fragment was isolated and ligated into the BamH1 site of M13mp18 (Messing et al., cited above). The recombinant phage, designated φRIT20001 was then used for mutagenesis experiments via the Amersham "Oligonucleotide--Directed in vitro Mutagenesis Systems", supra.

To delete amino acid 26 (tryptophan), i.e., Trp²⁶ of subunit S1, the following synthetic oligonucleotide primer was used: 5' CGTTGTTTCCCGCCGTGAAT 3' After mutagenesis, the site-specific mutant was sequenced to ensure that the correct mutation had been introduced. The double stranded replicative form of the phage DNA was then isolated and digested with AccI. The 300 bp DNA fragment, containing the mutation in S1, was then isolated and ligated into the AccI site of pRIT13070 (cited above).

A similar procedure was used to introduce single amino acid deletions or substitutions of subunit S1 at positions 9(Arg), 13(arg), 35 (His), 40(Ser), and 129(Glu). The single mutations in the S1 subunit are listed in Table 1 below (mutations # 11 to 22).

### Example 4. Combination of mutations

The pRIT13070 derived plasmids from Example 1 (i.e., S2 mutants) are digested with XbaI and XmaI to yield 750 bp fragments which encode S2 mutations. The XbaI - XmaI fragments will replace the XbaI - XmaI fragment from pRIT13070 derived plasmids of Example 3 (i.e., S1 mutants) to yield a recombinant DNA molecule having mutations in subunits S1 and S2; e.g., Trp²⁶(S1) - Asn¹⁰⁵(S2). In a similar manner, the pRIT13070 derived plasmids of Example 2 (i.e., S3 mutants) can be digested with BglII and BamHT. The DNA fragments then isolated (approximately 1,750 bp) will replace the BglII - BamHT fragment from pRIT13070 derived plasmids of Example 3 (i.e., S1 mutants) to yield mutations in subunits S1 and S3; e.g., Trp²⁶(S1) - Tyr¹⁰²⁻¹⁰³(S3). Furthermore, one skilled in the art is thus enabled to create additional combinations of S1, S1-S2, S1-S3, S2-S3, or S1-S2-S3 mutations based on this disclosure. The various combinations of mutations are listed in Table 1 below (mutations # 10 and 23 to 31).

### Example 5. Deletion of the toxin qene of a B. pertussis host

To express a pertussis toxin gene encoding one or more of the described mutations, it is desirable to first delete the wild-type B. pertussis toxin gene from the host cell. Thereby the toxin gene encoded by a plasmid will not recombine with the toxin gene encoded on the bacterial chromosome. Thus, the pertussis toxin coding sequence of the B. pertussis Tohama I vaccine strain was deleted in the following way:

The B. pertussis Tohama I strain (Sato, et al., in Manclark et al. (eds.), International Symposium on Pertussis, pp. 51-57, U.S. Department of Health, Education and Welfare, Washington, D.C. (1979), or Kasuga et al., Kitasato Arch. Exp. Med. 27:57-62) was plated on BG medium (BG agar, Difco Laboratories (Detroit, MI), supplemented with defibrinated sheep blood 25% v/v) containing 400 µg/ml of streptomycin. The streptomycin resistant mutants were then plated on BG medium containing 50 µg/ml nalidixic acid. The colonies that arose, which were streptomycin and nalidixic acid resistant were then used for conjugation experiments.

Plasmid pTOX9 (Black et al., Infect Immun, 55:2465-70 (1987)) contains the pertussis toxin gene on an approximately 10 kbp pertussis DNA fragment. Plasmid pTOX9 was digested with the restriction enzymes KpnI and BglII, then treated with Ba131 exonuclease, and finally religated. This resultant plasmid, in which the pertussis toxin gene was deleted, was subsequently digested with ClaI. This yielded an approximately 7 kbp fragment containing the flanking regions of a deleted pertussis toxin gene. This ClaI-ClaI fragment was end-filled, isolated and then ligated into the HindIII (end-filled) site of plasmid pSORTP1. Plasmid pSORTP1 was derived from plasmid pRTPl (Stibitz et al., Gene, 50:133-140 (1986)) in which the gentamicin gene has been inserted. Hence, pSORTP1 contains the gene for gentamicin resistance as well as the gene which confers sensitivity to streptomycin. Plasmid pSORTP1 does not autonomously replicate in B. pertussis, and therefore must recombine with the B. pertussis chromosome.

Prior to recombination, plasmid pSORTP1 containing the nucleotide sequences flanking the pertussis toxin gene, was introduced into E. coli strain SM10 (Simon et al., BioTechnology, 1:784-791 (1983)). Several colonies were grown, and the plasmid DNA from each of those clones was individually analyzed by DNA sequence analysis to identify and verify the deletions.

An E. coli SM10 clone containing the recombinant plasmid pSORTP1 was conjugated with the wild-type B. pertussis Tohama I strain (disclosed above). This strain was streptomycin resistant (a recessive trait) and nalidixic acid resistant. The B. pertussis exconjugates were then plated onto BG agar plates containing nalidixic acid and gentamicin. The gentamicin resistant colonies, which were also streptomycin sensitive, could arise only if the entire recombinant plasmid was integrated into the B. pertussis chromosome. The gentamicin resistant clones were then plated onto BG plates containing 400 µg/ml streptomycin to select for a second recombination event resulting in streptomycin. resistant B. pertussis revertants. These revertants arose due to a second homologous recombination between the chromosome and the inserted plasmid. Many of the resulting streptomycin resistant strains which were gentamicin sensitive again had lost the wild-type pertussis toxin via the recombination event. The colonies were analyzed by Southern blot hybridization to verify the loss of the wild-type pertussis toxin gene. The resulting B. pertussis strain, which lacks the pertussis toxin gene, was then used for the expression of mutated pertussis toxin genes.

### Example 6(a). Expression of the Modified Pertussis Toxin Genes in B. pertussis with a replicating plasmid

The pRIT13070 derived plasmids containing the described mutations in the S2 or S3 subunits were digested with the restriction enzyme EcoRI. The resulting 4.7 kbp fragments were ligated into the unique EcoRI site of plasmid pLAFRII (Friedman et al., Gene*,* 18:289-296 (1982)). Plasmid pLAFRII contains a tetracycline resistance gene and therefore transformants with this plasmid can be selected on growth medium containing 12.5 µg/ml tetracycline. The recombinant plasmids were then introduced into E. coli SM10. The tetracycline resistant transformants were further analyzed by DNA sequencing for the presence of the mutated toxin genes. The colonies containing the desired mutations were conjugated with the B. pertussis strain, deleted in the pertussis toxin gene, as described in Example 5. Tetracycline resistant B. pertussis strains were then grown in modified Stainer-Scholte medium (Difco) containing 10 µg/ml tetracycline. The cells were then separated from the culture medium by centrifugation. The cells and the culture medium were both analyzed by Western blot using pertussis toxin specific monoclonal antibodies (see Francotte et al. in Vaccine 89, p. 243-247, Lerner et al., eds., Cold Spring Harbor Laboratory, Cold Spring Harbor, NY, 1989 and Frank et al., Infect Immun, 46:195-201 (1984)).

For comparison, the wild-type B. pertussis strain (Tohama I) produced and secreted pertussis toxin into the culture media. The B. pertussis strain in which the pertussis toxin (PT) gene was deleted did not express a pertussis toxin. However, when this strain was transformed with a wild-type PT gene, carried by pLAFRII (supra), the PT was expressed at comparable, albeit somewhat lower levels as determined ELISA as disclosed in Example 7. Clones containing the mutant pertussis toxin genes produced significant amounts, e.g., 1-4 µg/ml, of mutated toxin proteins which were shown to be secreted into the culture medium.

### Example 6(b). Expression of the modified pertussis toxin genes in B. pertussis with a plasmid integrated in the chromosome.

The pRIT 13070 derived plasmids containing the described mutations or combinations of these mutations were transformed by electroporation in B. pertussis deleted in the pertussis toxin gene (described in example 5). The electroporation was performed as follows. B. pertussis was grown in 10 ml of modified Stainer-Scholte medium till exponential phase. Cells were harvested by centrifugation, washed twice in ice-cold water and resuspended in 2 to 5 ml of water. 80 µl of this suspension and 2 µl of plasmid DNA were mixed and submitted to a single electrical pulse (2.5 MV, 200 Ω, 25 µF) in a 0.8 mm-gap cuvette electrode. The cells were directly transferred into 1 ml of medium, incubated at 37°C for 1 to 4 hours and spread onto Bordet-Gengou plates containing ampicillin. As the pRIT 13070 derived plasmids are unable to replicate into B. pertussis, the ampicillin resistance can only be acquired by the integration of the plasmid by homologous recombination between the plasmid and the chromosome.

Ampicillin resistant B. pertussis strains were grown in modified Stainer-Scholte medium supplemented with 50 µg/ml of ampicillin. The cells were then separated from the medium by centrifugation. The supernatant was then analyzed by Western blot and ELISA using pertussis toxin-specific monoclonal and polyclonal antibodies (supra). Clones containing the mutant pertussis toxin genes produced significant amounts (0.5 - 10 µg/ml) of mutated toxin protein secreted into the culture medium.

### Example 7. Biological Activity of the Modified Pertussis Toxin Polypeptides

The culture media of the various B. pertussis strains producing different genetically modified pertussis toxin proteins were analyzed by an enzyme-linked immunosorbent assay (ELISA) using haptoglobin-coated microtiter plates as described by Francotte et al. (supra). The absorbance was compared to the estimated concentration of the various toxin analogs in the culture medium. This analysis allowed identification of mutant proteins which possessed altered haptoglobin-binding activity. None of the mutations in the S3 subunit significantly altered the haptoglobin-binding ability of pertussis toxin. Mutations in the S2 subunit diminished the haptoglobin-binding capacity of pertussis toxin by various amounts. The mutant protein containing the deletion Tyr¹⁰² in subunit S2 bound to haptoglobin with approximately 50% efficiency. The binding capacity of the PT protein containing a double deletion (i.e., Tyr¹⁰²⁻¹⁰³) was reduced to approximately 4%, as compared to the wild type molecule; the haptoglobin-binding capacity of the PT protein containing the Asn¹⁰⁵ deletion was decreased to undetectable levels (i.e., less than 0.5% of the wild type activity). These results indicate that Asn¹⁰⁵ of the S2 subunit is essential for haptoglobin-binding. The analogous mutation in the S3 subunit (Lys¹⁰⁵) did not affect the haptoglobin-binding capacity, suggesting that the binding of the toxin to haptoglobin and haptoglobin-like receptors occurs specifically via Dimer 1 (i.e., subunits S2 and S4).

As indicated above, Dimer 2 (subunits S3 and S4) appears to specifically bind to CHO cell membranes (Brennan et al., supra). Therefore, pertussis toxin analogs containing alterations in the S3 subunit may affect this CHO-cell specific cytotoxicity. The mutant proteins analyzed in these examples exhibited reduction in cytotoxicity, and the deletions Tyr¹⁰²⁻¹⁰³ and Lys¹⁰⁵ in subunit S3 exhibited significant reduction in such cytotoxicity. For these deletions, i.e., Tyr¹⁰²⁻¹⁰³ and Lys¹⁰⁵, no cytotoxicity was detectable in the culture medium, indicating an apparent detoxification factor of at least 100 fold. It is further noted that all of the mutant proteins were recognized by monoclonal antibodies to the individual subunits.

### Example 8. Parenteral Vaccine preparation

5-25 µg of mutated toxin protein, prepared according to the method of Example 6, is mixed with an aluminum adjuvant, such as aluminum hydroxide, to produce a vaccine in a form appropriate for incorporation into a parenteral administration dosage form.

It is appreciated that the invention is not limited to the particular embodiments described above in the Examples. All embodiments of the invention, therefore, are believed to come within the scope of the following claims.

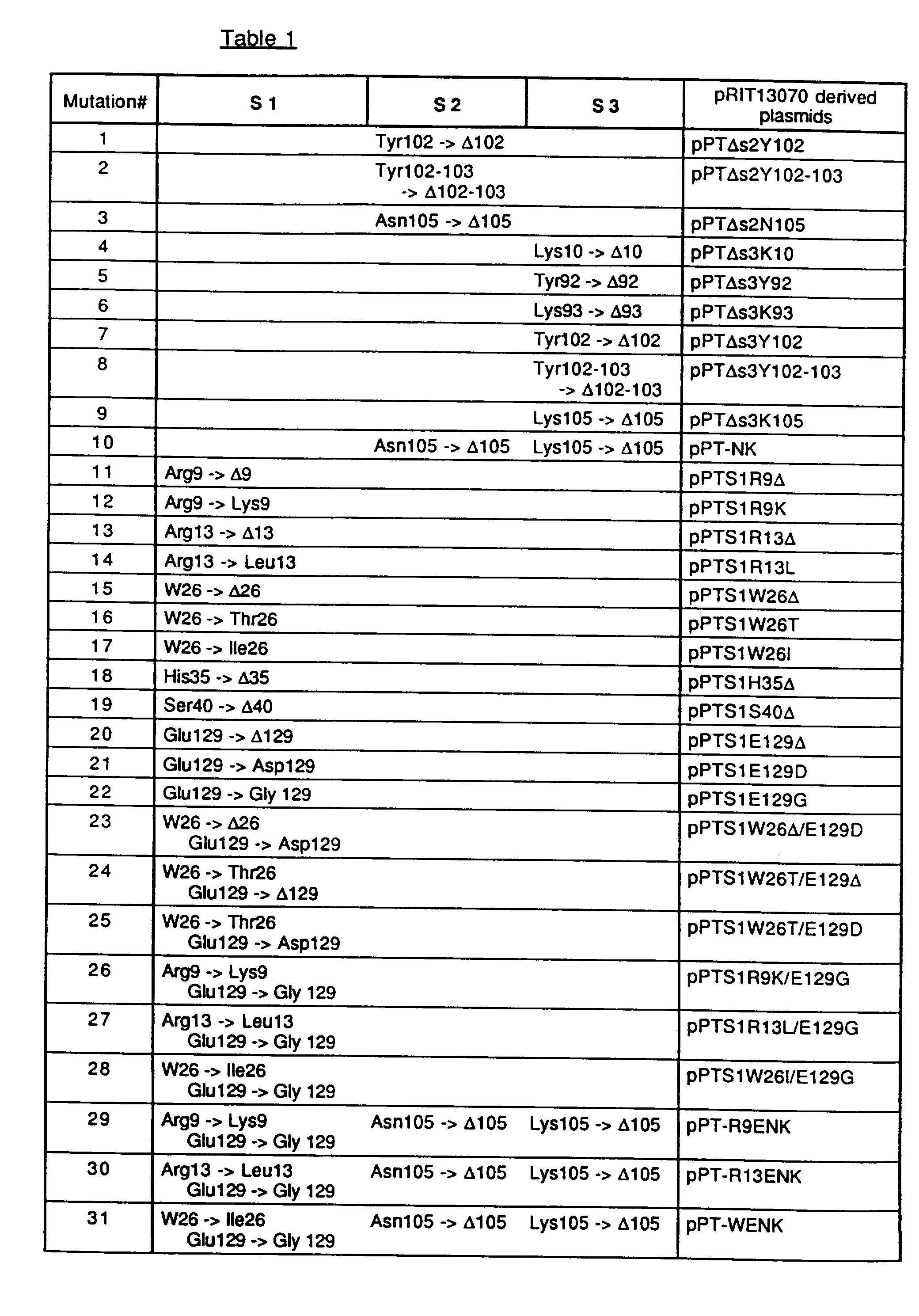

## Claims

1. A recombinant DNA molecule which comprises a DNA coding sequence which encodes a protein specifically reactive with antibodies against wild-type pertussis toxin but which is defective in pertussis toxin target cell receptor binding, characterised in that the recombinant DNA molecule encodes a Bordetella pertussis toxin B-oligomer and comprises a mutant dimer D1 coding sequence and/or a mutant dimer D2 coding sequence, subject to the proviso that in the absence of a mutant dimer D1 coding sequence the mutation in the D2 coding sequence is a deletion mutation.

2. The recombinant DNA molecule of claim I which encodes a mutant Bordetella pertussis toxin, further comprising a mutant or wild-type S1 subunit coding sequence.

3. The recombinant DNA molecule of claim 1 which encodes a mutant Bordetella pertussis toxin, comprising a mutation in the Bordetella pertussis toxin S2 subunit coding sequence, or in the S3 subunit coding sequence, or in both the S2 and the S3 subunit coding sequences, subject to the proviso that in the absence of a mutant S2 subunit coding sequence, the mutation in the S3 subunit coding sequence is a deletion mutation.

4. The recombinant DNA molecule of claim 1 which encodes a substitution or deletion mutation in the Bordetella pertussis toxin S2 subunit coding sequence.

5. The recombinant DNA molecule of claim 4 which encodes a substitution of Asn¹⁰⁵ to Asp¹⁰⁵

6. The recombinant DNA molecule of claim 1 which comprises a deletion of 1 to 5 amino acid codons from either or both of the S2 and S3 subunit coding sequences.

7. The recombinant DNA molecule of claim 6 in which a mutation in the S2 subunit coding sequence is selected from a deletion of the amino acid codon for Asn¹⁰⁵, Tyr¹⁰² and Tyr¹⁰²⁻¹⁰³ and/or a mutation in the S3 subunit coding sequence is selected from a deletion of the amino acid codon for Lys¹⁰⁵,Tyr¹⁰² and Tyr¹⁰²⁻¹⁰³.

8. The recombinant DNA molecule of claim 1 in which the Bordetella pertussis S1 subunit coding sequence comprises one or more mutations such that the mutant pertussis toxin encoded thereby is defective in ADP-ribosyltransferase activity.

9. The recombinant DNA molecule of claim 8 in which the mutation in the S1 subunit coding sequence is selected from a deletion in one or more of amino acids Arg⁹, or Arg¹³, or Trp²⁶, or His³⁵, or Ser⁴⁰, or Glu¹²⁹.

10. A recombinant plasmid which comprises the DNA molecule of claim 1 operatively linked to a regulatory region.

11. A host cell transformed with the recombinant plasmid of claim 10.

12. The host cell of claim 1 which is E. coli.

13. A host cell which contains the recombinant DNA molecule of claim 1 integrated into the host cell's genome.

14. The host cell of claim 13 which is Bordetella pertussis.

15. A whole cell vaccine for stimulating protection against whooping cough wherein such vaccine comprises an immunoprotective and non-toxic quantity of the inactivated host cell of claim 11.

16. A whole cell vaccine for stimulating protection against whooping cough wherein such vaccine comprises an immunoprotective and non-toxic quantity of the inactivated host cell of claim 13.

17. A protein encoded for by the recombinant DNA molecule of claim 1.

18. A vaccine for stimulating protection against whooping cough wherein such vaccine comprises an immunoprotective and non-toxic quantity of the protein of claim 17.

19. A process for preparing a protein encoded by the recombinant DNA molecule of claim 1 which comprises:
(a) introducing the recombinant DNA molecule into a host cell; and
(b) growing said host cell in a suitable culture medium.

20. The process of claim 19 wherein the host cell is deficient in the wild-type Bordetella pertussis toxin gene.

21. The process of claim 19 which further comprises isolating the protein so produced.

22. Use of the protein of claim 17 for the manufacture of a vaccine for protecting a human against disease symptoms associated with whooping cough infection.

23. Use of the vaccine of claim 18 for the manufacture of a medicament for protecting a human against disease symptoms associated with whooping cough infection.

## Patentansprüche

1. Rekombinantes DNA-Molekül, umfassend eine codierende DNA-Sequenz, die ein Protein codiert, das spezifisch mit Antikörpern gegen Wildtyp-Pertussis-Toxin reagiert, aber nicht an Rezeptoren auf Zielzellen von Pertussis-Toxin bindet, dadurch gekennzeichnet, daß das rekombinante DNA-Molekül ein B-Oligomer von Bordetella pertussis-Toxin codiert und eine mutierte codierende Sequenz des Dimers D1 und/oder eine mutierte codierende Sequenz des Dimers D2 umfaßt, mit der Maßgabe, daß in Abwesenheit einer mutierten codierenden Sequenz des Dimers D1 die Mutation in der D2 codierenden Sequenz eine Deletionsmutation ist.

2. Rekombinantes DNA-Molekül nach Anspruch 1, das ein mutiertes Bordetella pertussis-Toxin codiert, weiterhin umfassend eine mutierte oder Wildtyp-codierende Sequenz der S1-Untereinheit.

3. Rekombinantes DNA-Molekül nach Anspruch 1, das ein mutiertes Bordetella pertussis-Toxin codiert, umfassend eine Mutation in der die S2-Untereinheit von Bordetella pertussis-Toxin codierenden Sequenz oder in der die S3-Untereinheit codierenden Sequenz oder sowohl in der die S2- als auch in der die S3-Untereinheit codierenden Sequenz, mit der Maßgabe, daß in Abwesenheit einer mutierten codierenden Sequenz der S2-Untereinheit die Mutation in der die S3-Untereinheit codierenden Sequenz eine Deletionsmutation ist.

4. Rekombinantes DNA-Molekül nach Anspruch 1, das eine Substitutions- oder Deletionsmutation in der die 52-Untereinheit von Bordetella pertussis-Toxin codierenden Sequenz codiert.

5. Rekombinantes DNA-Molekül nach Anspruch 4, das eine Substitution von Asn¹⁰⁵ zu Asp¹⁰⁵ codiert.

6. Rekombinantes DNA-Molekül nach Anspruch 1, umfassend eine Deletion von 1 bis 5 Aminosäurecodons von den entweder eine oder beide S2- und S3-Untereinheiten codierenden Sequenzen.

7. Rekombinantes DNA-Molekül nach Anspruch 6, in dem eine Mutation in der die S2-Untereinheit codierenden Sequenz ausgewählt ist aus einer Deletion des Aminosäurecodons für Asn¹⁰⁵, Tyr¹⁰² und Tyr¹⁰²⁻¹⁰³ und/oder eine Mutation in der die S3-Untereinheit codierenden Sequenz ausgewählt ist aus einer Deletion des Aminosäurecodons für Lys¹⁰⁵, Tyr¹⁰² und Tyr¹⁰²⁻¹⁰³.

8. Rekombinantes DNA-Molekül nach Anspruch 1, wobei die die S1-Untereinheit von Bordetella pertussis codierende Sequenz eine oder mehrere Mutationen umfaßt, so daß das dadurch codierte mutierte Pertussistoxin keine ADP-Ribosyltransferaseaktivität aufweist.

9. Rekombinantes DNA-Molekül nach Anspruch 8, in dem die Mutation in der die S1-Untereinheit codierenden Sequenz ausgewählt ist aus einer Deletion in einer oder mehreren der Aminosäuren Arg⁹, Arg¹³, Trp²⁶, His³⁵, Ser⁴⁰ oder Glu¹²⁹.

10. Rekombinantes Plasmid, umfassend das DNA-Molekül nach Anspruch 1, in funktioneller Verknüpfung mit einem regulatorischen Bereich.

11. Wirtszelle, transformiert mit dem rekombinanten Plasmid nach Anspruch 10.

12. Wirtszelle nach Anspruch 11, die E. coli ist.

13. Wirtszelle, die das rekombinante DNA-Molekül nach Anspruch 1 integriert in das Wirtszellengenom enthält.

14. Wirtszelle nach Anspruch 13, die Bordetella pertussis ist.

15. Impfstoff aus ganzen Zellen zur Stimulation eines Schutzes gegen Keuchhusten, wobei der Impfstoff eine Immunschutz verleihende und nicht-toxische Menge der inaktivierten Wirtszelle nach Anspruch 11 umfaßt.

16. Impfstoff aus ganzen Zellen zur Stimulation eines Schutzes gegen Keuchhusten, wobei der Impfstoff eine Immunschutz verleihende und nicht-toxische Menge der inaktivierten Wirtszelle nach Anspruch 13 umfaßt.

17. Protein, das von dem rekombinanten DNA-Molekül nach Anspruch 1 codiert wird.

18. Impfstoff zur Stimulation des Schutzes gegen Keuchhusten, wobei der Impfstoff eine Immunschutz verleihende und nicht-toxische Menge des Proteins nach Anspruch 17 umfaßt.

19. Verfahren zur Herstellung eines Proteins, das von dem rekombinanten DNA-Molekül nach Anspruch 1 codiert wird, umfassend:
(a) Einführung des rekombinanten DNA-Moleküls in eine Wirtszelle, und
(b) Züchtung der Wirtszelle in einem geeigneten Kulturmedium.

20. Verfahren nach Anspruch 19, wobei die Wirtszelle kein Gen des Wildtyp-Bordetella pertussis-Toxins aufweist.

21. Verfahren nach Anspruch 19, weiterhin die Isolierung des so produzierten Proteins umfassend.

22. Verwendung des Proteins nach Anspruch 17 für die Herstellung eines Impfstoffes zum Schutz eines Menschen gegen Krankheitssymptome, die mit einer Keuchhusteninfektion verknüpft sind.

23. Verwendung des Impfstoffes nach Anspruch 18 für die Herstellung eines Medikaments zum Schutz eines Menschen gegen Krankheitssymptome, die mit einer Keuchhusteninfektion verknüpft sind.

## Revendications

1. Molécule d'ADN recombinante qui comprend une séquence codant pour un ADN qui code pour une protéine capable de réagir de façon spécifique avec des anticorps dirigés contre la toxine de pertussis de type sauvage, mais est déficiente en ce qui concerne la liaison avec un récepteur de cellule cible de toxine de pertussis, caractérisée en ce que la molécule d'ADN recombinante code pour un oligomère B de toxine de *Bordetella pertussis* et comprend une séquence codant pour un dimère D1 mutant et/ou une séquence codant pour un dimère D2 mutant, à la condition qu'en l'absence d'une séquence codant pour un dimère D1 mutant, la mutation dans la séquence codant pour D2 soit une mutation par délétion.

2. Molécule d'ADN recombinante suivant la revendication 1 qui code pour une toxine mutante de *Bordetella pertussis,* comprenant de plus une séquence codant pour une sous-unité S1 de type sauvage ou mutante.

3. Molécule d'ADN recombinante suivant la revendication 1 qui code pour une toxine mutante de *Bordetella pertussis,* comprenant une mutation dans la séquence codant pour la sous-unité S2 de toxine de *Bordetella pertussis,* ou dans la séquence codant pour la sous-unité S3, ou à la fois dans les séquences codant pour les sous-unités S2 et S3, à condition qu'en absence d'une séquence codant pour une sous-unité S2 mutante, la mutation dans la séquence codant pour la sous-unité S3 soit une mutation par délétion.

4. Molécule d'ADN recombinante suivant la revendication 1, qui code pour une mutation par substitution ou par délétion dans la séquence codant pour la sous-unité S2 de toxine de *Bordetella pertussis.*

5. Molécule d'ADN recombinante suivant la revendication 4, qui code pour une substitution de Asn¹⁰⁵ en Asp¹⁰⁵.

6. Molécule d'ADN recombinante suivant la revendication 1, qui comprend une délétion de 1 à 5 codons d'acides aminés de l'une ou de l'autre ou des deux séquences codant pour les sous-unités S2 et S3.

7. Molécule d'ADN recombinante suivant la revendication 6, dans laquelle une mutation dans la séquence codant pour la sous-unité S2 est choisie parmi une délétion du codon d'acide aminé pour Asn¹⁰⁵, Tyr¹⁰² et Tyr¹⁰²⁻¹⁰³ et/ou une mutation dans la séquence codant pour la sous-unité S3 est choisie parmi une délétion du codon d'acide aminé pour Lys¹⁰⁵, Tyr¹⁰² et Tyr¹⁰²⁻¹⁰³.

8. Molécule d'ADN recombinante suivant la revendication 1, dans laquelle la séquence codant pour la sous-unité S1 de *Bordetella pertussis* comprend une ou plusieurs mutations si bien que la toxine mutante de pertussis ainsi codée est déficiente en activité ADP-ribosyltransférase.

9. Molécule d'ADN recombinante suivant la revendication 8, dans laquelle la mutation dans la séquence codant pour la sous-unité S1 est choisie parmi une délétion d'un ou de plusieurs des acides aminés Arg⁹, ou Arg¹³, ou Trp²⁶, ou His³⁵, ou Ser⁴⁰ ou Glu¹²⁹.

10. Plasmide recombinant qui comprend la molécule d'ADN suivant la revendication 1, reliée de façon opérative à une région régulatrice.

11. Cellule hôte transformée avec le plasmide suivant la revendication 10.

12. Cellule hôte suivant la revendication 11, qui est *E. coli.*

13. Cellule hôte qui contient la molécule d'ADN recombinante suivant la revendication 1 intégrée dans le génome de la cellule hôte.

14. Cellule hôte suivant la revendication 11, qui est *Bordetella pertussis.*

15. Vaccin à cellule entière pour stimuler une protection contre la coqueluche, dans lequel ce vaccin comprend une quantité immunoprotectrice et non toxique de la cellule hôte inactivée suivant la revendication 11.

16. Vaccin à cellule entière pour stimuler une protection contre la coqueluche, dans lequel ce vaccin comprend une quantité immunoprotectrice et non toxique de la cellule hôte inactivée suivant la revendication 13.

17. Protéine codée par la molécule d'ADN recombinante suivant la revendication 1.

18. Vaccin pour stimuler une protection contre la coqueluche, dans lequel ce vaccin comprend une quantité immunoprotectrice et non toxique de la protéine suivant la revendication 17.

19. Procédé pour la préparation d'une protéine codée par la molécule d'ADN recombinante suivant la revendication 1, qui comprend :
(a) l'introduction de la molécule d'ADN recombinante dans une cellule hôte; et
(b) la croissance de cette cellule hôte dans un milieu de culture convenable.

20. Procédé suivant la revendication 19, dans lequel la cellule hôte est déficiente en gène de toxine de *Bordetella pertussi* de type sauvage.

21. Procédé suivant la revendication 19, qui comprend de plus l'isolement de la protéine ainsi produite.

22. Utilisation de la protéine suivant la revendication 17 pour la fabrication d'un vaccin pour protéger un humain contre des symptômes de maladie associés à une infection par la coqueluche.

23. Utilisation du vaccin suivant la revendication 18 pour la fabrication d'un médicament pour protéger un humain contre des symptômes de maladie associés à une infection par la coqueluche.
